# EUROPEAN PATENT APPLICATION

(11) **EP 2 343 101 A1**
(43) Date of publication of application: **13.07.2011**
(21) Application number: 10703212.0
(22) Date of filing: 15.01.2010
(51) Int. Cl.: A61M 37/00, B29C 59/02, B81B 1/00, B81C 1/00

(54) **STAMPER FOR MICRONEEDLE SHEET, METHOD FOR MANUFACTURING THE STAMPER, AND METHOD FOR MANUFACTURING MICRONEEDLE USING THE STAMPER**

(30) Priority: 08.10.2009 JP 2009234109
(71) Applicant: BioSerenTach Co., Ltd., Kyoto 600-8040 (JP)
(72) Inventor: MOTOI, Masashi, (JP); HONDA, Kenshin, (JP); TERADA, Toyoharu, (JP); OKADA, Tatsuya, (JP); SATOUCHI, Yoshitomo, (JP); TAKADA, Kanji, (JP)
(74) Representative: Haley, Stephen
(86) International application number: PCT/JP2010/050407
(87) International publication number: WO 2011/043085

(57) **Abstract**

Microneedle sheets are produced by injecting a needle raw material into a stamper formed with a concavity in a base material. However, because the stamper concavity is very small, there is the problem that air cannot escape due to air and surface tension of the material, thereby preventing a needle from being formed in part. The present invention provides a stamper for producing a microneedle sheet. The stamper comprises a sheet-like base material including a conical concavity formed from a first surface of the sheet-like base material toward a second surface thereof; and a through-hole formed from a bottom of the concavity toward the second surface. Air can escape via this through-hole, allowing a microneedle sheet which is filled with a needle raw material as far as the bottom of the concavity to be obtained.

## Description

### TECHNICAL FIELD

The present invention relates to a microneedle sheet for injecting a drug into the epidermis of a skin. More specifically, the present invention relates to a stamper, which is a mold, for producing a microneedle, and a method for producing a microneedle sheet using this stamper.

### BACKGROUND ART

A microneedle sheet is a sheet in which very small needles having a length of about 1 µm to 500 µm, and a high aspect ratio of the cross-sectional diameter at the base to the length of cross-sectional diameter:length = 1:1.5 to 1:3, are arranged at a predetermined density onto a sheet substrate. The microneedle sheet is used for injecting a drug by placing it against mainly a skin portion of a human body, and inserting the microneedles (hereinafter, sometimes referred to simply as "needle") into the epidermis portion in the skin.

Since the microneedles have a length of about several hundred µm, they cause almost no pain or itchiness. Furthermore, the microneedle portion is formed from a self-dissolving substance so that when the sheet is removed from the skin there is no harm to the body even if the microneedles remain in the skin.

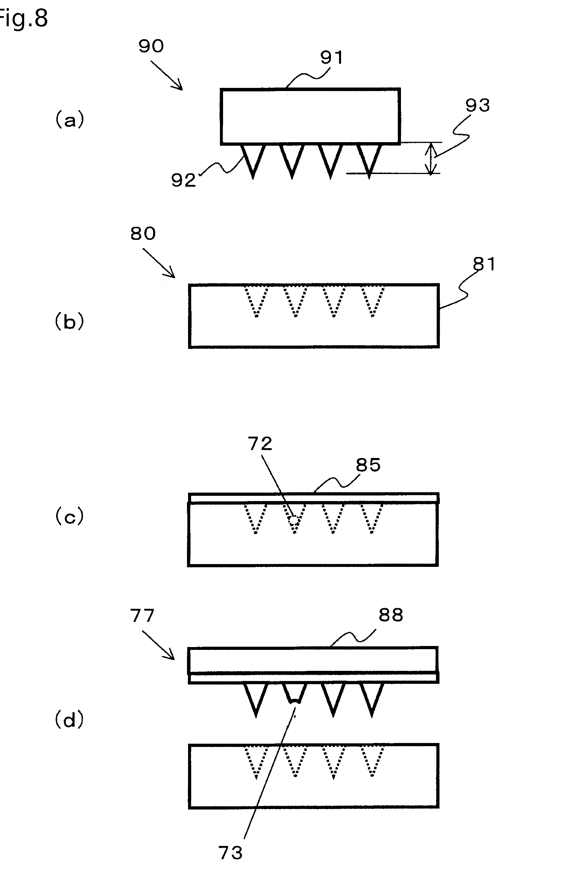

A common method for producing a microneedle sheet will now be described with reference to Fig. 8. An original plate 90 of a microneedle sheet is produced by a method such as fine machining, a vacuum treatment, or photolithography. As described above, the needle has a length 93 of several hundred µm or less, and has a conical shape. The needle cross-section may be circular, angular, elliptical or the like. Since the needle has a very small size, it is preferred to form the needle 92 by shaving from an original plate sheet 90.

Next, the original plate 90 is pressed against a stamper base material 81 to produce a stamper 80 (a mold for a microneedle sheet). Then, a resin polymer solution or a drug 85 is injected into the stamper 80. The resin polymer solution or drug 85 is dried, then stuck onto a fixing substrate 88 to be transferred thereonto and peeled off from the stamper 80, whereby a microneedle sheet 77 can be obtained (see Patent Document 1).

In the method for producing this stamper 80, rather than pressing the original plate 90 against the base material 81, the dissolved resin may be injected into the original plate 90, dried, and then peeled off (see Patent Document 2).

### LIST OF PRIOR ART DOCUMENTS

### PATENT DOCUMENTS

[Patent Document 1] Japanese Patent Application Laid-Open No. 2008-245955
[Patent Document 2] Japanese Patent Application Laid-Open No. 2008-006178
[Patent Document 3] Japanese Patent Application Laid-Open No. 2009-083125

### SUMMARY OF THE INVENTION

### PROBLEMS TO BE SOLVED BY THE INVENTION

In either case, the microneedle sheet is produced by injecting a raw material of the needle into a produced stamper. However, since the concavities in the stamper have extremely small dimensions, the raw material of the microneedles is not easily filled into the stamper concavities due to air and surface tension of the material.

More specifically, in Fig. 8(c), when a resin polymer solution or a drug 85 is injected into the stamper 81, air 72 is present in a concavity of the stamper. When peeling from the stamper, at a portion (73) where the air 72 was present, the needle can only be formed in an incomplete shape (Fig. 8(d)).

This problem is also described in Patent Documents 2 and 3. Patent Document 2 describes carrying out the filling of the raw material into the stamper under a reduced pressure. Patent Document 3 describes filling a raw material into a stamper concavity while applying a pressure from the raw material side. However, when providing many microneedles, there is the problem that the raw material is not filled in some of the concavities, which means that not all of the planned microneedles can be planted.

### MEANS FOR SOLVING THE PROBLEMS

The present invention was created in consideration of the above-described problems. Specifically, the present invention provides a stamper for producing a microneedle sheet formed on a fixing substrate, the stamper comprising a sheet-like base material including:
a conical concavity formed from a first surface of the sheet-like base material toward a second surface thereof; and
a through-hole formed from a bottom of the concavity toward the second surface. Furthermore, the present invention provides a method for producing the stamper according to the present invention, and a method for producing a microneedle sheet using the inventive stamper.

### ADVANTAGES OF THE INVENTION

In the present invention, a through-hole which penetrates as far as an opposite face of a stamper is provided on the bottom of a concavity of the stamper. Therefore, air can easily escape even if air has accumulated in the concavity of the stamper. Consequently, the present invention can obtain a microneedle sheet on which all of the planned microneedles are formed by enabling a raw material to be filled into all of the concavities of the stamper.

### BRIEF DESCRIPTION OF THE DRAWINGS

Fig. 1 is a schematic diagram of a stamper according to the present invention.
Fig. 2 is a diagram illustrating a production process of the stamper according to the present invention.
Fig. 3 is a diagram illustrating another production process of the stamper according to the present invention.
Fig. 4 is a diagram illustrating yet another production process of the stamper according to the present invention.
Fig. 5 is a diagram illustrating a production process of a microneedle sheet using the stamper according to the present invention.
Fig. 6 is a diagram illustrating a production process of a microneedle sheet using the stamper according to the present invention.
Fig. 7 is a diagram illustrating another production process of a microneedle sheet using the stamper according to the present invention.
Fig. 8 is a diagram illustrating a production process of a microneedle sheet using a conventional stamper.

### BEST MODE FOR CARRYING OUT THE INVENTION

### (Embodiment 1)

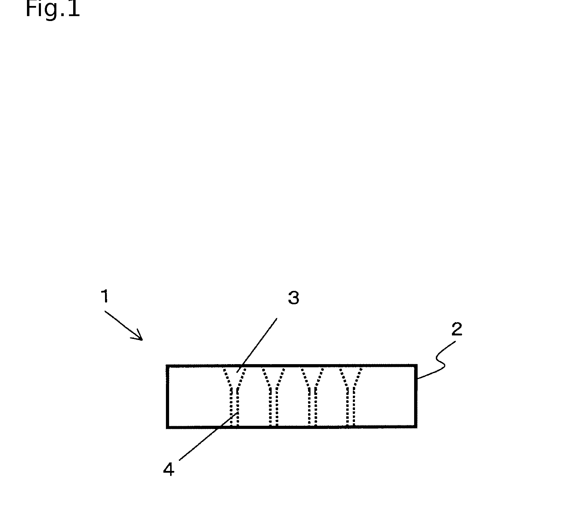

Fig. 1 illustrates a stamper 1 of a microneedle sheet according to the present invention. The stamper 1 is formed with conical concavities 3 in a sheet-like base material 2. The material of the base material 2 is not especially limited. However, considering that such a material will be used together with a medicine, a material which does not easily contaminate or does not have an effect on the human body is preferred. Examples of metals which may be preferably used include SUS 316L and hastelloy. Examples of plastics which may be preferably used include PTFE, polypropylene, and polyethylene. A through-hole 4 which penetrates to another face of the base material is formed in each of bottom portions of the concavities 3. The through-hole 4 does not have to be a hole with a finite length which is constantly open, so long as air which has accumulated in the concavity can escape in order for the microneedle raw material to be filled into the concavities 3. More specifically, the through-hole 4 may be formed by a through-cut which, even though it normally looks like it is closed, allows air to escape when the raw material is being filled.

Next, examples of the method for producing the stamper according to the present invention will be described. However, the present invention is not limited to these production methods, so long as the gist of the present invention is not departed from.

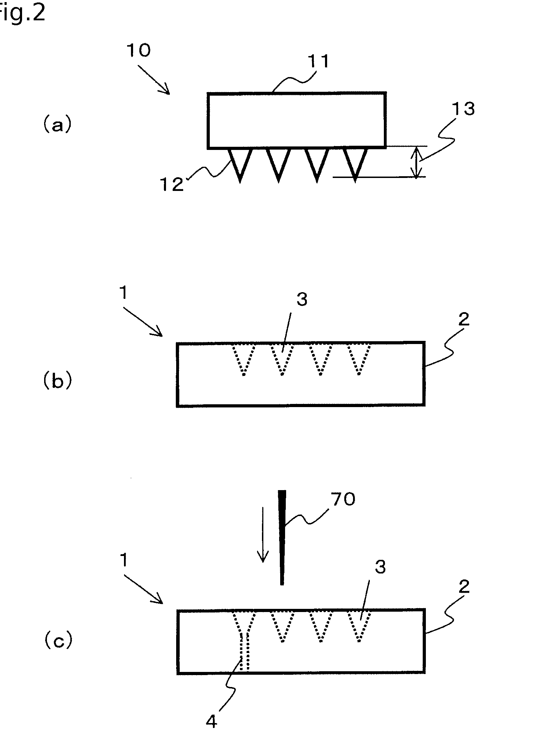

With reference to Fig. 2(a), an original plate 10 includes a flat body 11 on which conical protrusions 12 are formed. The material for the original plate 10 is not especially limited, but it is preferred to use a metal which has excellent workability. Since the conical protrusions are very small, and have a different cross-section at the base and the tip, a material which can be easily shaved is preferred. Examples of the material used include copper, aluminum, nickel, silicon and the like. In addition to the processing of the protrusions 12 by shaving the protrusions from the body 11, the protrusions 12 may also be formed by a method using photolithography.

The protrusions 12 stand on the body 11 with a height 13 of from about 1 µm to 500 µm. The reason for the height setting is as follows. Although the epidermis into which the drug or the like is injected using the microneedle sheet is formed from the stratum corneum, stratum granulosum, stratum spinosum, and stratum basale, the thickness of the stratum corneum is different depending on the area of the body. Thus, the height of the protrusions can change depending on the area of the body into which transdermal administration is to be carried out and the drug.

The protrusions 12 have a needle shape with a comparatively high aspect ratio of the cross-sectional diameter at the base to the length of cross-sectional diameter:length = 1:1.5 to 1:3. This is because the aspect ratio may change depending on the area to be used. The cross-sectional shape is not especially limited. Examples of shapes which may be preferably used include circular, elliptical, rectangular including square, and the like. Furthermore, a groove may be formed on the surface of the protrusions. This is because a microneedle which reflects the shape of such a groove can be smoothly inserted into the skin.

Next, with reference to Fig. 2(b), the original plate 10 is pressed against the base material 2 of the stamper 1 to form conical concavities 3 on the surface of the base material. As described above, an organic compound (plastic) may be mainly used for the base material 2. Since a through-hole is formed from the surface of the base material toward the back face, thickness selection is important in the present invention. The thickness is preferably thicker than the depth of the concavities 3 to be formed in the stamper 1 by 10 µm or more to 50 µm or less. This is because the through-holes cannot be formed after the formation of the concavities if the thickness is too thick.

Although the organic compound mainly used for the base material 2 undergoes partial plastic deformation when the conical protrusions 12 are compressed from the surface, an elastic deformed portion also remains. Accordingly, if the protrusions 12 are stripped off, the concavities 3 can become smaller than the shape which has been pushed wider by the protrusions 12 of the original plate. Therefore, it is preferred for the protrusions 12 of the original plate to be produced with a size which is larger than the concavities of the stamper that are to be produced.

Next, with reference to Fig. 2(c), a through-hole 4 is formed in the bottom of the concavities 3 of the stamper 1. The through-holes can be opened by a micro drill 70. The micro drill may also form the through-holes by allowing needle-shaped protrusions that do not have a spiral blade to perforate the stamper.

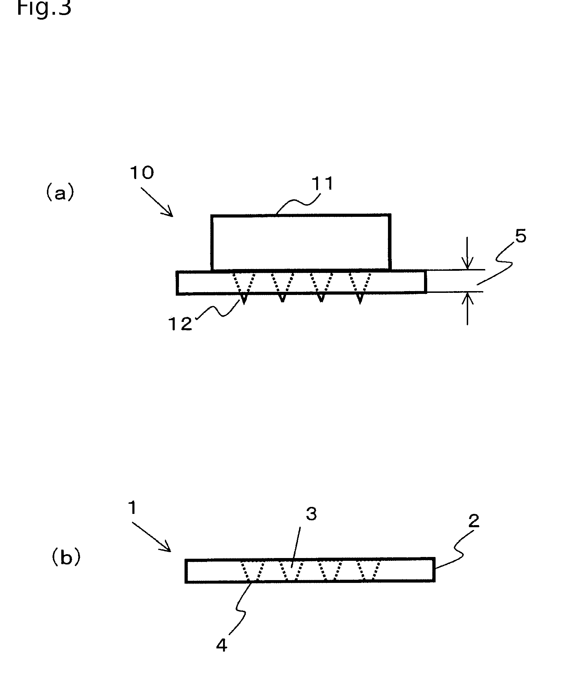

From this perspective, as illustrated in Fig. 3(a), the through-holes may also be formed by pre-setting a thickness 5 of the substrate to be slightly thinner than the protrusions 12 of the original plate, so that the substrate is perforated by the original plate protrusions when the original plate is pressed against, and brought in contact with, the substrate. In this case, the step of pressing the original plate against the substrate and bringing in contact with and the step of forming the through-holes are carried out simultaneously. Consequently, as illustrated in Fig. 3(b), the stamper 1 has through-holes 4 whose cross-sectional area changes from the surface of the base material 2 toward the back face thereof.

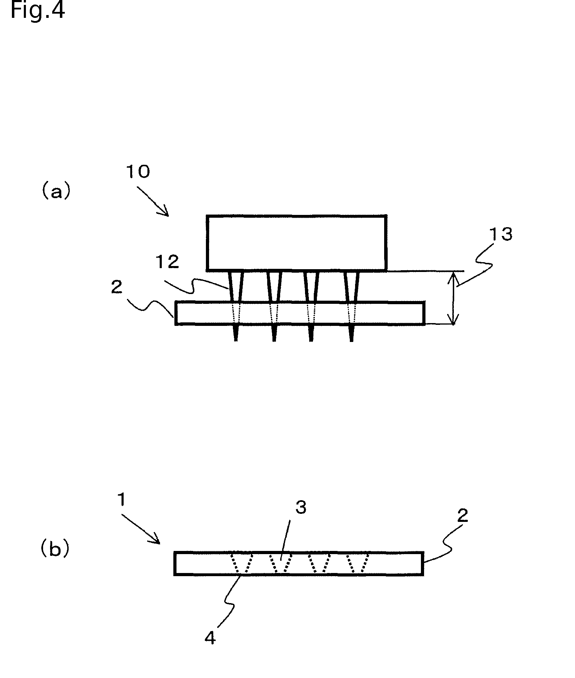

Fig. 4 illustrates a case in which the protrusions 12 of the original plate 10 and the stamper are pre-heated. Since the base material 2 of the stamper is a plastic, plastic deformation can be easily made to occur if the protrusions 12 are heated to a temperature higher than a melting point of the base material 2. In this case, when the protrusions 12 are inserted into and then released from the base material 2, the surface portion of the base material is heated and deforms. Consequently, it is preferred to release the original plate and the stamper after cooling to room temperature.

Even when the protrusions 12 of the original plate 10 are pre-heated, the through-holes may be formed after the concavities have been formed to midway through the thickness of the base material 2. Alternatively, the concavities and the through-holes may be simultaneously formed by making the protrusions 12 of the original plate 10 penetrate as far as the back face of the base material 2. Fig. 4 illustrates a case in which the stamper is formed by this latter method.

As illustrated in Fig. 4, when making the protrusions 12 of the original plate 10 penetrate the base material 2, the protrusions 12 of the original plate 10 are not reflected as is in the shape of the microneedles. Therefore, the stamper 1 is produced so that the ratio of the cross-sectional diameter at the base to the length of the microneedles produced by the stamper 1 produced as illustrated in Fig. 4 has an aspect ratio of cross-sectional diameter:length = 1:1.5 to 1:3.

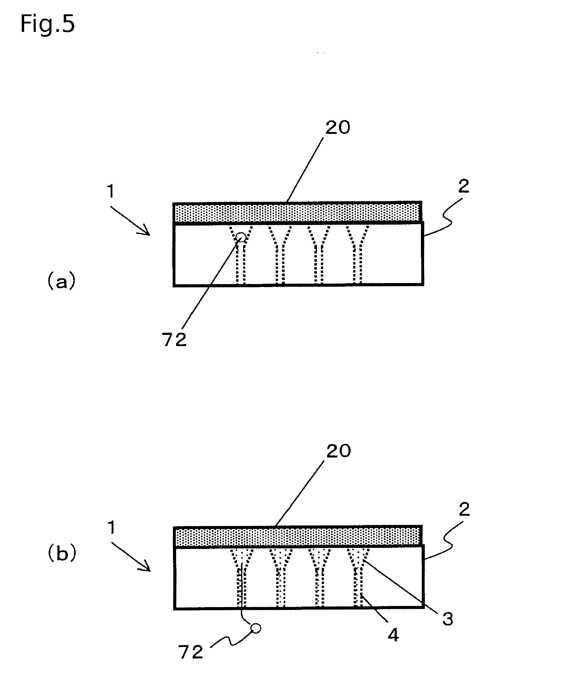

The microneedle sheet is produced using the thus-produced stamper. With reference to Fig. 5, the microneedle sheet is produced by coating a needle raw material 20 on the stamper 1 (Fig. 5(a)). As the needle raw material 20, it is preferred to use a material which is discharged without remaining in the body. The reason for this is as follows. When the microneedle sheet is peeled off from the skin, the needle portions may break or be pulled out of the sheet, so that some needles cannot be recovered. Therefore, in such cases, it is safer if the material is absorbed by the body. Specifically, the needle raw material is preferably a material which has a thread-forming substance and the like as a main component. It is also preferred to pre-mix the drug in the needle raw material. Here, the term "drug" refers to a pure chemical substance having a physiologically active effect. Examples of such a substance include peptide/protein drugs such as insulin, growth hormone, erythropoietin, and interferon, polymer drugs, vitamin C and the like. The drug may also be a substance having any one of them as a main component.

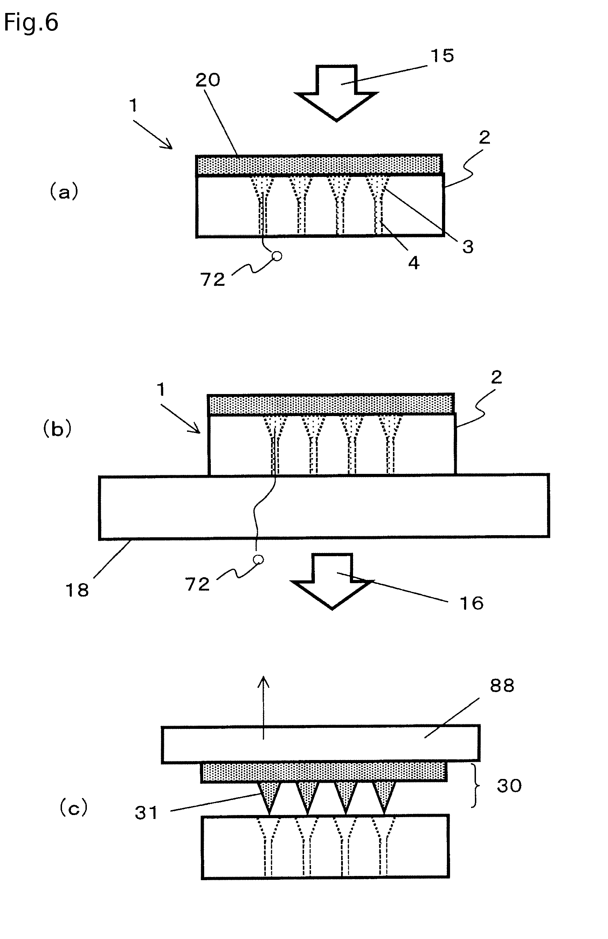

As illustrated in Fig. 5(b), the stamper 1 according to the present invention has the through-holes 4 formed in the bottoms of concavities 3. Therefore, to inject the needle raw material into the stamper, the needle raw material is filled into the concavities just by coating. Specifically, even if air 72 is present at the bottoms of the concavities 3 of the stamper 1, the air can easily be discharged via the through-holes 4, so that the needle raw material is filled into the concavities 3. Obviously, a pressure 15 may be applied after the coating (Fig. 6(a)). If a pressure is applied, air which has accumulated at the bottoms of the concavities 3 of the stamper can easily escape.

Furthermore, the stamper 1 may be arranged on a flat mount 18 formed by a porous solid, and the needle raw material 20 can be coated while applying a negative pressure 16 from the back face of the flat mount 18 (see Fig. 6(b)). In this case too, the needle raw material can be easily injected into the concavities 3 of the stamper. Moreover, it is more preferred to apply a pressure 15 from the top face of the flat mount 18 and apply the negative pressure 16 from the back face.

After the needle raw material 20 is coated on the stamper 1, the needle raw material 20 is dried and peeled from the stamper, thereby completing a microneedle sheet 30 (see Fig. 6(c)). The peeling from the stamper 1 is carried out by sticking the fixing substrate 88 from the back face of the dried needle raw material 20, and peeling from the stamper. If a drug was not added into the microneedle sheet 30 peeled from the stamper, after being peeled from the stamper, the drug is dispersed or the like in a needle portion 31.

Thus, in the stamper according to the present invention, a through-hole which penetrates as far as an opposite face of the stamper is machined into the bottom of a concavity forming a needle. Therefore, even if air is in the concavity, the air can easily escape, which allows a microneedle sheet which has a high degree of completeness, namely, which has no incomplete needles, to be obtained.

### (Embodiment 2)

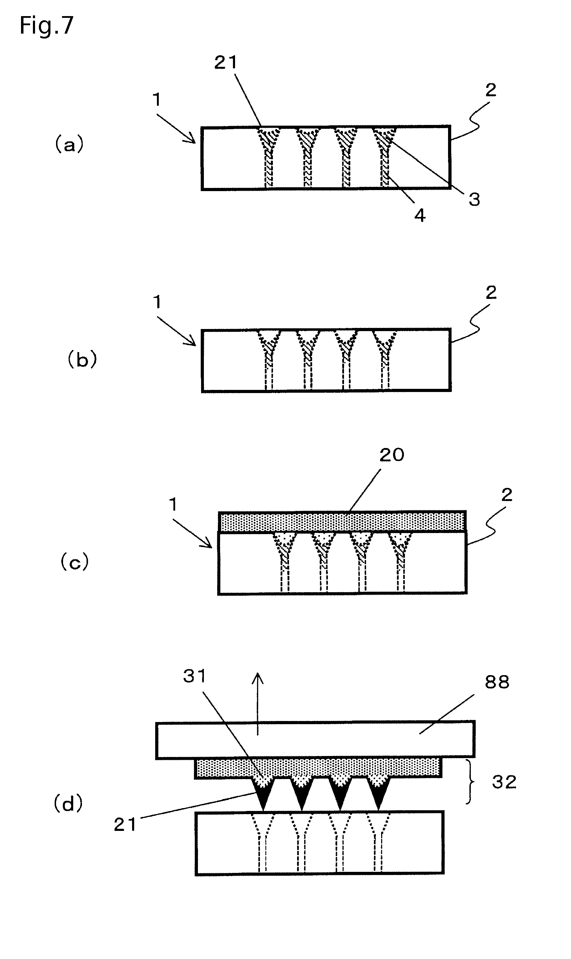

Fig. 7 illustrates another example of a microneedle sheet. Embodiment 2 is the same as Embodiment 1 up to the method for producing the stamper. In the case of mixing the drug into the needle raw material, the drug is also dispersed in the sheet substrate portion. However, although the substrate portion basically touches the skin surface, the drug is not injected into the epidermis. Accordingly, in the present embodiment, the drug is concentrated in the needle tip portion.

First, a drug 21 is coated on the stamper (see Fig. 7(a)). At this stage, the drug 21 is coated to about a level where it accumulates in the bottom portions of the concavities 3 of the stamper 1. The coating is carried out by coating a diluted solution of the drug on the stamper. It is preferred to subsequently scrape off excess solution with a squeegee or the like. Obviously, pressure or a negative pressure from the back face may be applied.

The drug solution 21 is dried, and then stays in the inner faces and the bottom portions of the concavities 3 (see Fig. 7(b)). Next, the needle raw material 20 is coated (see Fig. 7(c)). Since the needle raw material does not directly contact the stamper material at the concavity portions of the stamper, the needle raw material can be thoroughly coated so that air does not accumulate.

Then, after scraping off the excess drug solution with a squeegee, the needle raw material is immediately coated, and pressure or a negative pressure from the back face may be applied. In this case, the coating is wet-on-wet coating. Therefore, similar to Embodiment 1, air escapes from the through-holes just by coating, so that a microneedle sheet can be obtained which includes the drug in the tips.

Finally, the fixing substrate 88 is stuck to the back face of the dried needle raw material, and peeled from the stamper 1. Here, the obtained microneedle sheet 32 is a microneedle sheet in which the drug 21 is provided in the tips of the needles.

In the present embodiment, the microneedle sheet was formed from two layers, a layer including the drug and a layer forming the needle. However, the structure can have three or more layers.

### INDUSTRIAL APPLICABILITY

The present invention can not only be used for a microneedle sheet which injects a drug into the epidermis, but can also be widely used as a method for generating very small protrusions on a substrate.

### DESCRIPTION OF REFERENCE NUMERALS

- 1: stamper
- 2: base material
- 3: concavity

- 4: through-hole
- 5: thickness of base material
- 10: original plate
- 11: body of original plate
- 12: protrusion
- 13: height of protrusion
- 15: pressure
- 16: negative pressure
- 18: flat mount made of porous solid
- 20: needle raw material
- 21: drug
- 30, 32: microneedle sheet
- 31: needle
- 70: micro drill
- 72: air
- 77: microneedle sheet
- 80: conventional stamper
- 81: conventional stamper base material
- 85: needle raw material
- 88: fixing substrate
- 90: original plate
- 91: body
- 92: protrusion
- 93: length of protrusion

## Claims

1. A stamper for producing a microneedle sheet formed on a fixing substrate, the stamper comprising a sheet-like base material including:
a conical concavity formed from a first surface of the sheet-like base material toward a second surface thereof; and
a through-hole formed from a bottom of the concavity toward the second surface.

2. A method for producing a stamper for a microneedle sheet formed on a fixing substrate, the method comprising the steps of:
forming a conical concavity from a first surface of a sheet-like base material toward a second surface of the base material; and
forming a through-hole from a bottom of the concavity toward the second surface.

3. The method for producing a stamper according to claim 2,
wherein
the step of forming a concavity is a step of pressing an original plate having a conical convexity against the first surface of the sheet-like base material and bringing it into contact with the first surface, and wherein
the step of forming a through-hole is a step of perforating the second surface of the base material with a tip of the convexity of the original plate pressed against and brought into contact with the base material.

4. The method for producing a stamper according to claim 3, wherein the step of pressing the original plate against the first surface and bringing it into contact with the first surface is a step of preparing the base material made of a resin, heating the original plate and pressing the heated original plate against the base material and bringing it into contact with the base material.

5. A method for producing a microneedle sheet formed on a fixing substrate, the method comprising the steps of:
forming a conical concavity from a first surface of a sheet-like base material toward a second surface of the base material;
forming a through-hole from a bottom of the concavity toward the second surface to obtain a sheet-like stamper;
injecting a needle raw material from the first surface of the stamper; and
sticking a fixing substrate on the first surface.

6. The method for producing a microneedle sheet according to claim 5, wherein
the step of injecting a needle raw material includes a step of first injecting a first needle raw material and then a step of injecting at least a second needle raw material.

7. The method for producing a microneedle sheet according to any of claims 5 and 6, wherein
the step of injecting the needle raw material is a step of injecting while applying a pressure to the stamper from the first surface side.

8. The method for producing a microneedle sheet according to any of claims 5 and 6, wherein
the step of injecting the needle raw material is a step of injecting while sucking the stamper from the second surface side.
